# EUROPÄISCHE PATENTSCHRIFT

(11) **EP 1 407 520 B1**
(45) Veröffentlichungstag und Bekanntmachung des Hinweises auf die Patenterteilung: **29.03.2006**
(21) Anmeldenummer: 02753024.5
(22) Anmeldetag: 17.07.2002
(51) Int. Cl.: G02B 27/09, A61F 9/01

(54) **STRAHLFORMUNGSELEMENT FÜR OPTISCHE STRAHLUNG SOWIE VERFAHREN ZUR HERSTELLUNG**
BEAM-SHAPING ELEMENT FOR OPTICAL RADIATION AND A METHOD FOR PRODUCING SAID ELEMENT
ELEMENT DE FORMATION DE RAYONNEMENT POUR RAYONNEMENT OPTIQUE ET SON PROCEDE DE PRODUCTION

(30) Priorität: 18.07.2001 DE 10134893
(43) Veröffentlichungstag der Anmeldung: 14.04.2004
(73) Patentinhaber: Fraunhofer-Gesellschaft zur Förderung der angewandten Forschung e.V., 80686 München (DE)
(72) Erfinder: QUENZER, Hans-Joachim, 25524 Itzehoe (DE); REINERT, Wolfgang, 24536 Neumünster (DE)
(74) Vertreter: Gagel, Roland
(86) Internationale Anmeldenummer: PCT/DE2002/002617
(87) Internationale Veröffentlichungsnummer: WO 2003/009432

(56) Entgegenhaltungen:
- EP-A- 1 191 360
- WO-A-01/28478
- US-A- 5 961 861
- US-A- 6 061 323

## Beschreibung

### Technisches Anwendungsgebiet

Die vorliegende Erfindung betrifft ein Strahlformungselement für optische Strahlung, insbesondere für UV-Laserstrahlung, mit einem Trägersubstrat, das eine Dünnschicht-Metallisierung trägt, die zur Strahlformung auftreffender optischer Strahlung strukturiert ist. Die Erfindung betrifft weiterhin ein Verfahren zur Herstellung eines Strahlformungselementes für optische Strahlung.

Strahlformungselemente werden bspw. zur Strahlformung von Laserstrahlung für Anwendungen im Bereich der Materialbearbeitung oder auch der medizinischen Technik eingesetzt. Die Strahlformungselemente dienen dabei in erster Linie zur Erzeugung eines vorgebbaren Intensitätsprofils der Laserstrahlung, das für die jeweilige Anwendung von Vorteil ist. So können Strahlformungselemente bspw. zur Abflachung des Intensitätsprofils intensiver UV-Laserstrahlung eingesetzt werden, um bei einer flächigen Materialbearbeitung einen stufenlosen Übergang zu unbearbeiteten Bereichen erzeugen zu können. Das bearbeitete Material kann hierbei auch belebter Natur sein. Gerade im medizinischen Bereich, bspw. bei der Bearbeitung der Hornhaut des menschlichen Auges mit einem UV-Laser, ist ein derartiger stufenloser Übergang zur Korrektur von Hornhautfehlern erforderlich. Das Strahlformungselement ist hierbei als mikrostrukturierte Strahlformungsblende ausgebildet, an deren Mikrostrukturen das eintreffende Intensitätsprofil der Laserstrahlung gebeugt wird, um ein gewünschtes flaches Strahlprofil zu erhalten. Bei derartigen Strahlformungselementen ist eine dünne metallische Schicht auf einem transparenten Trägersubstrat aufgebracht, in der die Mikrostrukturen erzeugt werden.

Gerade beim Einsatz intensiver UV-Laserstrahlung wird jedoch diese Metallisierung mit der Zeit durch die Strahlung beschädigt, so dass die Wirkung des Strahlformungselementes auf die eintreffende Laserstrahlung nicht mehr vorhersehbar ist. Dies kann gerade im Bereich der medizinischen Technik zu erheblichen Gefährdungen für den Patienten führen, da der Verschleiß der Metallisierung für den Benutzer anfänglich nicht erkennbar ist. Aus diesem Grunde sollten im Bereich der medizinischen Technik Strahlformungselemente für intensive UV-Strahlung jeweils nur einmalig benutzt werden. Um diese einmalige Benutzung sicherzustellen, ist jedoch ein sehr sorgfältiges Arbeiten des Benutzers erforderlich, da dem Strahlformungselement eine einmalige Benutzung in der Regel nicht angesehen werden kann. Es kann daher vorkommen, dass ein bereits benutztes Strahlformungselement versehentlich mehrfach eingesetzt wird.

Der nächste Stand der Technik, WO 0128478, offenbart ein Strahlformungselement mit einem An-Ti-Cn (80nm-40nm-80nm) coating. Es wird vorgeschlagen dieses Element nach einmaligem Gebrauch auszuwesechseln und gebrauchte Strahlformungselemente durch eine zusätzliche Laserbestrahlung oder elektronisch zu markieren.

Die Aufgabe der vorliegenden Erfindung besteht darin, ein Strahlformungselement für optische Strahlung, insbesondere für UV-Laserstrahlung, sowie ein Verfahren zur Herstellung eines Strahlformungselementes anzugeben, an dem beim Einsatz in intensiver optischer Strahlung eine einmalige Benutzung erkennbar ist.

### Darstellung der Erfindung

Die Aufgabe wird mit dem Strahlformungselement und dem Verfahren gemäß den Patentansprüchen 1 bzw. 6 gelöst. Vorteilhafte Ausgestaltungen des Strahlformungselementes sowie des Verfahrens sind Gegenstand der Unteransprüche.

Das vorliegende Strahlformungselement aus einem Trägersubstrat, das eine strukturierte Dünnschicht-Metallisierung zur Strahlformung der optischen Strahlung trägt, zeichnet sich dadurch aus, dass sich die Dünnschicht-Metallisierung aus zumindest zwei metallischen Schichten zusarmnensetzt, die beim Auftreffen der optischen Strahlung ab einer bestimmten Intensität und/oder Bestrahlungsdauer der optischen Strahlung unter permanenter Farbänderung miteinander reagieren, wobei eine obere Schicht der beiden metallischen Schichten aus An mit einer Schichtdicke von 100nm und eine untere Schicht aus Cr mit einer Schichtdicke von 40nm gebildet wird, und die Gesamtschichtdicke der Dünnschicht 140nm beträgt.

Die Reaktion der Materialien der beiden metallischen Schichten führt vorzugsweise zu einer Legierungsbildung der beteiligten Metalle, die durch eine durch die eintreffende optische Strahlung hervorgerufene Erwärmung dieser Schichten an der Auftreffstelle der optischen Strahlung ausgelöst wird. Durch diese Legierungsbildung wird bei geeigneter Wahl der beteiligten Metalle eine deutliche Farbänderung gegenüber der Farbe der oberen metallischen Schicht hervorgerufen. Dem Fachmann sind geeignete Metalle für eine derartige Legierungsbildung unter Farbänderung geläufig. Die erforderliche Energie für die Auslösung der Reaktion sollte hierbei selbstverständlich deutlich oberhalb der ohne Einstrahlung der optischen Strahlung vorliegenden Wärmeenergie liegen. Weiterhin darf die Intensität bzw. Bestrahlungsdauer mit der optischen Strahlung, bei der die Reaktion ausgelöst wird, nicht derart hoch liegen, dass bei dieser Intensität und/oder Bestrahlungsdauer bereits eine Beschädigung der metallischen Schichten eintritt.

Bei Einsatz des vorliegenden Strahlformungselementes zur Formung des Intensitätsprofils optischer Strahlung, insbesondere von Laserstrahlung, die eine ausreichende Intensität aufweist bzw. mit einer ausreichenden Bestrahlungsdauer eingestrahlt wurde, kann durch den Benutzer sofort nach diesem Einsatz anhand einer an der Auftreffstelle der optischen Strahlung erfolgten Farbänderung erkannt werden, dass das Strahlformungselement bereits einmal eingesetzt wurde. Gerade im Bereich der medizinischen Technik beim Einsatz von intensiver UV-Laserstrahlung können auf diese Weise Fehler durch eine Mehrfachverwendung des Strahlformungselementes verhindert werden. Die durch die Farbänderung an der Auftreffstelle hervorgerufenen Gebrauchsspuren deuten einen möglichen Verschleiß der Metallisierung optisch an, lange bevor die Metallisierung Risse oder Löcher durch Strahlungsablation aufweist. Trotz dieser optisch sichtbaren Gebrauchsspuren bleibt die Funktionalität der Metallisierung des Strahlformungselementes weiterhin erhalten, so dass zu diesem Zeitpunkt noch keine Risse oder Haftungsprobleme in der Metallisierung auftreten.

Erfindungsgemäß werden die metallischen Schichten beim vorliegenden Strahlformungselement derart gewählt, dass sich die Reflektivität der Dünnschicht-Metallisierung für die eingesetzte optische Strahlung durch die hervorgerufene Reaktion beider Schichten permanent erniedrigt. Durch diese nochmals verringerte Reflektion im verfärbten Bereich verlängert sich die Lebensdauer der vorgeschalteten hochwertigen Optik, die insbesondere bei Einsatz von UV-Laserstrahlung durch zu hohe Reflektion am Strahlformungselement leicht beschädigt werden kann.

Erfindungsgemäß ist die obere der beiden metallischen Schichten aus Gold mit einer Schichtdicke von 100nm gebildet. Die Verwendung dieser edlen Metalldeckschicht erlaubt unbefristete Lagerzeiten der Strahlformungselemente, so dass auch hierdurch keine Fehlfunktion ausgelöst werden kann. Die untere der beiden metallischen Schichten, d. h. die auf dem Trägersubstrat aufliegende Schicht, ist aus Chrom mit einer Schichtdicke von 40nm gebildet. Chrom hat in diesem Zusammenhang zahlreiche Vorteile. So kann es zum einen als Haftvermittler zwischen dem Trägersubstrat und der oberen Schicht dienen. Andererseits lässt sich dieses Material sehr gut nasschemisch ätzen, um die erforderliche Mikrostrukturierung für die Strahlformung zu erzeugen.

Die Dünnschicht-Metallisierung bestehend aus den zumindest zwei metallischen Schichten muss selbstverständlich so dick gewählt werden, dass die für die Strahlformung erforderliche optische Dichtheit erreicht wird. Vorzugsweise hat diese Dünnschicht-Metallisierung jedoch eine Gesamtschichtdicke von ≤ 250 nm, so dass eine mikrooptische Strukturierung mit Strukturbreiten unterhalb von 250 nm erreicht werden kann.

Bei dem vorliegenden Verfahren zur Herstellung eines Strahlformungselementes für optische Strahlung, insbesondere eines Strahlformungselementes gemäß der vorangegangenen Beschreibung, wird zunächst eine untere Schicht aus Chrom auf ein für die optische Strahlung transparentes Trägersubstrat aufgebracht. Anschließend wird - ggf. nach dem Aufbringen einer Zwischenschicht - eine obere Schicht aus einem Edelmetall über der unteren Schicht aufgebracht. Die entstandene Schichtfolge wird dann für die erforderliche Strahlformung strukturiert. Die Strukturierung erfolgt durch Mikrostrukturierung der oberen Schicht mittels Photolithographie und einem anschließenden Trockenätzprozess, der bis an die untere Schicht ausgeführt wird. Die obere Schicht dient dann als Ätzmaske für die Mikrostrukturierung der unteren Schicht, die durch ein nasschemisches Ätzverfahren erfolgt. Auf diese Weise lässt sich die Strahlformungsblende in einfacher Weise mit einer exakten Mikrostruktur herstellen. Die untere und die obere Schicht werden vorzugsweise mit einem physikalischen Sputterverfahren aufgebracht.

Das vorliegende Verfahren ermöglicht die Herstellung eines Strahlformungselementes, wie es in der vorangehenden Beschreibung erläutert wurde.

### Kurze Beschreibung der Zeichnungen

Die vorliegende Erfindung wird nachfolgend ohne Beschränkung des allgemeinen Erfindungsgedankens anhand von Ausführungsbeispielen in Verbindung mit den Zeichnungen nochmals kurz erläutert. Hierbei zeigen:
- Fig. 1: erfindungsgemäß einen Aufbau des Strahlformungselementes der vorliegenden Erfindung im Querschnitt;
- Fig. 2: ein Beispiel für die Strahlformung des Strahlprofils von Laserstrahlung durch ein Strahlformungselement;
- Fig. 3: ein Beispiel für eine Gebrauchsspur, wie sie bei Einsatz des vorliegenden Strahl formungselementes erkennbar ist;
- Fig. 4: einen erfindungsgemäßen Verfahrensablauf für die Herstellung eines Strahlformungselementes gemäß Fig. 1; und

### Wege zur Ausführung der Erfindung

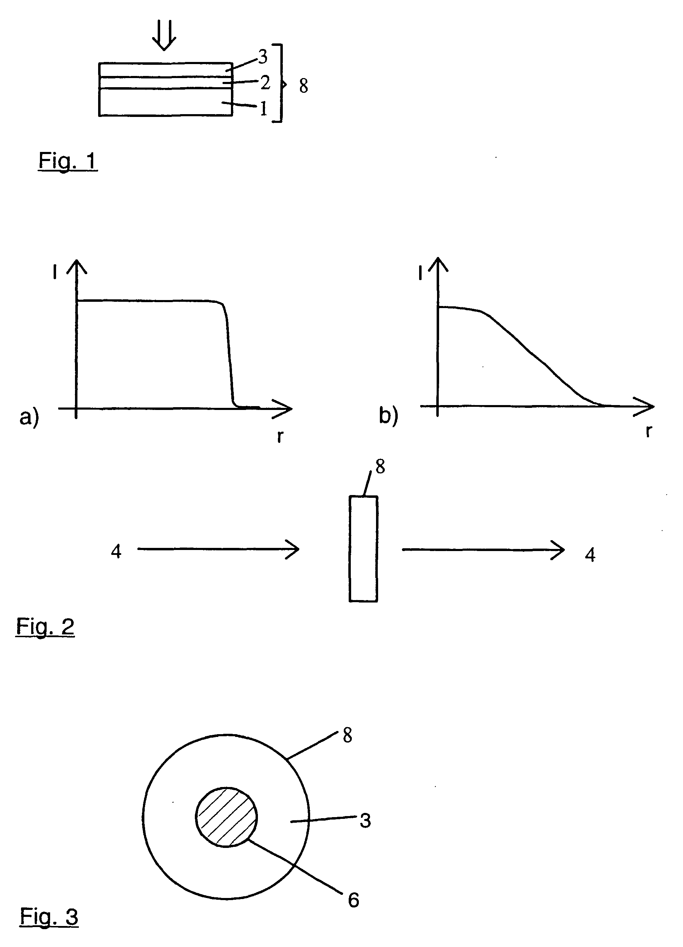

Figur 1 zeigt schematisch einen Aufbau eines erfindungsgemäßen Strahlformungselementes 8, wie es mit der vorliegenden Erfindung realisiert wird. Das Strahlformungselement 8 setzt sich in diesem Beispiel aus dem Trägersubstrat 1 mit einer darauf befindlichen Dünnschicht-Metallisierung aus zwei metallischen Schichten 2, 3 zusammen. Als Trägersubstrat 1 wird in diesem Beispiel doppelseitig poliertes Quarzglas verwendet, da dieses im Bereich der eingesetzten UV-Strahlung, insbesondere im Bereich von 193 nm, eine sehr hohe Transmission aufweist. Als untere Schicht 2 dieses Strahlformungselementes ist eine Chrom-Schicht aufgebracht, die als Haftvermittler der oberen Schicht 3 zum Quarzsubstrat 1 dient. Die obere metallische Schicht 3 besteht aus Gold, das bei der in diesem Beispiel eingesetzten Laserwellenlänge von 193 nm eine niedrige Reflektivität von ca. 10% aufweist. Die Gesamtschichtdicke der Dünnschicht-Metallisierung beträgt erfindungsgemäß 140 nm, wobei die untere Schicht 2 aus Chrom eine Dicke von 40 nm und die obere Schicht 3 aus Gold eine Dicke von 100 nm aufweist. Diese Schichtdicken lassen eine Mikrostrukturierung der Dünnschicht-Metallisierung mit Strukturbreiten bis zu 250 nm zu, die jedoch in dieser Figur nicht zu erkennen ist.

Figur 2 zeigt in Abbildung a) beispielhaft ein Intensitätsprofil eines UV-Laserstrahls 4, das mit dem Strahlformungselement 8 verändert werden soll. Das Intensitätsprofil hat eine annähernd rechteckige Form, so dass ein starker Intensitätsabfall an den Strahlbegrenzungen vorliegt, der in vielen Anwendungen bei der Materialbearbeitung unerwünscht ist. Das Strahlformungselement 8 gemäß der vorliegenden Erfindung kann dabei derart mikrostrukturiert sein, dass das Intentsitätsprofil des auftreffenden und hindurchtretenden UV-Laserstrahls 4 abgeflacht wird und einen stufenlosen flachen Verlauf an den Strahlbegrenzungen aufweist, wie dies beispielhaft in Teilabbildung b) zu erkennen ist.

Die für eine derartige Strahlformung erforderliche Geometrie der Mikrostrukturen der Dünnschicht-Metallisierung kann vorab Berücksichtigung optischer Gesetzmäßigkeiten berechnet werden.

Bei Einsatz des Strahlformungselementes 8, wie es bspw. in Figur 1 dargestellt ist, zur Strahlformung eines intensiven UV-Laserstrahls bei einer Wellenlänge von 193 nm, wie er bspw. im medizinischen Bereich bei Augenoperationen eingesetzt wird, verbleiben nach der ersten Benutzung deutlich sichtbare Gebrauchsspuren auf der Oberfläche der Dünnschicht-Metallisierung. Figur 3 zeigt beispielhaft ein derartiges Strahlformungselement 8 in Draufsicht auf die Dünnschicht-Metallisierung. Durch den Beschuss mit der UV-Laserstrahlung erfolgt am Auftreffort 6 eine lokale metallurgische Reaktion zwischen dem Gold und dem Chrom, die zu einer optisch sichtbaren Verfärbung der Oberfläche von gold-glänzend (Au) zu matt-grau-braun (Au-Cr-Legierung) führt. Diese Verfärbung tritt nur an der Auftreffstelle 6 der Laserstrahlung auf, während im restlichen Bereich der Oberfläche die reine Gold-Schicht mit ihrer goldglänzenden Farbe verbleibt. Auf diese Weise kann jederzeit sofort erkannt werden, ob das Strahlformungselement 8 bereits einmal benutzt wurde. Die optisch sichtbare Verfärbung führt im vorliegenden Fall zu einer gegenüber dem Anfangszustand deutlich verringerten Reflektivität für die UV-Laserstrahlung von unter 1%. Trotz der optisch sichtbaren Gebrauchsspuren bleibt die Funktionalität der Metallisierung weiterhin lange erhalten, so dass weder Risse noch Haftungsprobleme in der Metallisierung auftreten.

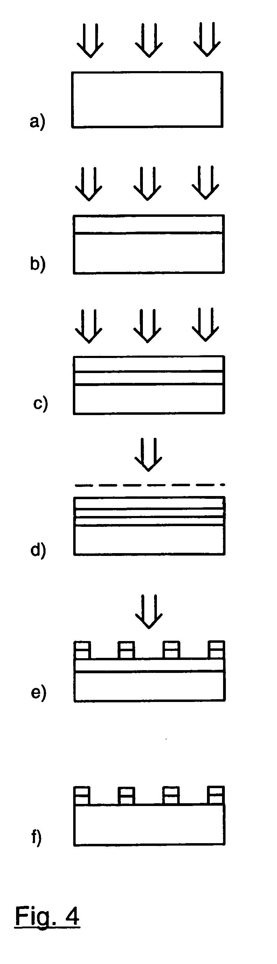

Figur 4 zeigt ein Beispiel für unterschiedliche Herstellungsschritte zur Herstellung eines Strahlformungselementes 8 bzw. einer Strahlformungsblende gemäß der Figur 1. Bei diesem Verfahren wird auf einem doppelseitig polierten Quarzwafer (Trägersubstrat 1) geringer Oberflächenrauhigkeit < 5 nm eine 40 nm dicke Cr-Schicht 2 durch physikalisches Aufsputtern aufgebracht (Figur 4 a/b). Ohne Unterbrechung des Vakuums wird nachfolgend ebenfalls durch physikalisches Aufsputtern eine 100 nm dicke Au-Schicht 3 auf die Cr-Schicht 2 aufgebracht. Hierbei wird eine sehr gute Schichthaftung erzielt (Figur 4 a/b). Anschließend wird ein lichtempfindlicher Photolack 7 auf die aus beiden Schichten 2, 3 gebildete Dünnschicht-Metallisierung aufgebracht (Figur 4 c/d). Durch ein Projektionssystem wird die Lackschicht 7 zur Erzeugung der gewünschten Mikrostrukturierung mit Hilfe einer Maske selektiv belichtet. Nach der Entwicklung des Photolackfilmes 7 wird die Goldschicht 3 in den exponierten Bereichen mit einem Argon-Plasmaätzer abgetragen und die Chromschicht 2 exponiert (vgl. Figur 4 e/f). Mit einer nasschemischen Chromätzlösung wird die Chromschicht 2 an den exponierten Stellen bis auf das Quarzsubstrat 1 abgeätzt, ohne das Quarz aufzurauhen. Die strukturierte Goldschicht 3 dient hierbei als Ätzmaske für den nasschemischen Ätzprozess. Nach der Entfernung des Photolackes 7 kann der Quarzwafer mit einer Diamanttrennschleifeinrichtung in einzelne Strahlformungselemente 8 zerteilt werden.

### Bezugszeichenliste

- 1: Trägersubstrat
- 2: untere metallische Schicht
- 3: obere metallische Schicht
- 4: optische Strahlung; Laserstrahlung
- 5: Zwischenschicht
- 6: Auftreffstelle
- 7: Photolack
- 8: Strahlformungselement

## Patentansprüche

1. Strahlformungselement für den Einsatz von UV-Laserstrahlung in der medizinischen Technik mit einem Trägersubstrat (1), das eine Dünnschicht-Metallisierung trägt, die zur Strahlformung der optischen Strahlung strukturiert ist und die sich aus zwei metallischen Schichten (2, 3) zusammensetzt, die beim Auftreffen der optischen Strahlung (4) ab einer bestimmten Intensität und/oder Bestrahlungsdauer der optischen Strahlung (4) unter permanenter Farbänderung miteinander reagieren,
**dadurch gekennzeichnet,**
**dass** eine obere Schicht (3) der beiden metallischen Schichten aus Au mit einer Schichtdicke von 100 nm und eine untere Schicht (2) aus Cr mit einer Schichtdicke von 40 nm gebildet sind und die Gesamtschichtdicke der Dünnschicht-Metallisierung 140 nm beträgt.

2. Strahlformungselement nach Anspruch 1,
**dadurch gekennzeichnet,**
**dass** sich die Reflektivität der Dünnschicht-Metallisierung für die optische Strahlung (4) durch die Reaktion permanent erniedrigt.

3. Strahlformungselement nach Anspruch 1 oder 2,
das für eine Wellenlänge der optischen Strahlung (4) von 193 nm ausgebildet ist.

4. Strahlformungselement nach einem der Ansprüche 1 bis 3,
**dadurch gekennzeichnet,**
**dass** das Trägersubstrat (1) aus Quarzglas besteht.

5. Strahlformungselement nach einem der Ansprüche 1 bis 4,
**dadurch gekennzeichnet,**
**dass** die Dünnschicht-Metallisierung zur Bildung einer optischen Blende für die auftreffende optische Strahlung (4) strukturiert ist.

6. Verfahren zur Herstellung eines Strahlformungselementes für optische Strahlung mit einem Trägersubstrat (1), der eine Dünnschicht-Metallisierung trägt und die sich aus zwei metallischen Schichten (2, 3) zusammensetzt nach den vorangehenden Patentansprüchen, mit zumindest folgenden Schritten:
- Aufbringen einer unteren Schicht (2) aus Cr auf ein für die optische Strahlung (4) transparentes Trägersubstrat (1),
- Aufbringen einer oberen Schicht (3) aus Au über der unteren Schicht (2), wobei die obere Schicht (3) aus Au mit einer Schichtdicke von 100 nm und die untere Schicht (2) aus Cr mit einer Schichtdicke von 40 nm aufgebracht werden, wobei die Gesamtschichtdicke der Dünnschichtmetallisierung 140nm beträgt,
- Mikrostrukturierung der oberen Schicht (3) mittels Photolithographie und einem anschließenden Trockenätzprozess, und
- Mikrostrukturierung der unteren Schicht (2) durch ein Nassätzverfahren, bei dem die obere Schicht (3) als Ätzmaske dient.

7. Verfahren nach Anspruch 6,
**dadurch gekennzeichnet,**
**dass** die untere (2) und die obere Schicht (3) mit einem physikalischen Sputterverfahren aufgebracht werden.

## Claims

1. A beam-shaping element for the use of UV-Laser-radiation in medical technology having a carrier substrate (1) bearing a thin-layer metallization which is structured for shaping the beam of said optical radiation and which is composed of two metallic layers (2, 3), which, upon impingement of the optical radiation (4), react with each other accompanied by a permanent change in color as of a certain intensity and/or irradiation period of the optical radiation (4),
**characterized in that** an upper layer (3) of both meallic layers is formed of Au with a thickness of 100nm, a lower layer (2) is formed of chrome with a thickness of 40nm, and the overall thickness of the thin layer is 140nm.

2. A beam-shaping element according to claim 1,
**characterized in that** the reaction permanently lowers the reflectivity of the thin-layer metallization for the optical radiation (4).

3. A beam-shaping element according to claim 1 or 2,
**characterized in that** the beam-shaping element is designed for a wavelength of the optical radiation (4) of 193 nm.

4. A beam-shaping element according to one of the claims 1 to 3, **characterized in that** said carrier substrate (1) is made of quartz glass.

5. A beam-shaping element according to one of the claims 1 to 4, **characterized in that** said thin-layer metallization is structured to form an optical diaphragm for said impinging optical radiation (4).

6. A method for producing a beam-shaping element for optical radiation having a carrier substrate (1) bearing a thin-layer metallization which is composed of two metallic layers (2, 3), according to the preceding claims, comprising at least the following steps:
- application of a lower layer (2) of Cr on a carrier substrate (1) which is transparent for said optical radiation (4),
- application of an upper layer (3) of Au over said lower layer (2), whereby the upper layer (3) consisting of Au with a thickness of 100nm and the lower layer (2) of chrome with a thickness of 40nm are applied and the overall thickness of the thin layer is 140nm,
- microstructuring of the upper layer (3) are applied by means of photolithography and a subsequent dry etching process, and
- microstructuring of the lower layer (2) by means of a wet-etching process, in which the upper layer (3) acts as an etching mask.

7. A method according to claim 6,
**characterized in that** the lower layer (2) and the upper layer (3) are applied using a physical sputtering method.

## Revendications

1. Elément de formage de rayons pour l'utilisation de rayonnement laser UV en technique médicale, comprenant un substrat porteur (1) comportant une métallisation en couche mince qui est structurée pour le formage de rayons du rayonnement optique et qui est composé de deux couches métalliques (2, 3) qui, lors de l'incidence du rayonnement optique (4), à partir d'une certaine intensité et/ou durée d'irradiation du rayonnement optique (4), réagissent entre elles en changeant de couleur en permanence,
**caractérisé en ce que**
la couche supérieure (3) des deux couches métalliques est composée d'Au d'une épaisseur de couche de 100 nm et la couche inférieure (2) de Cr d'une épaisseur de couche de 40 nm et que l'épaisseur de couche globale de la métallisation en couche mince s'élève à 140 nm.

2. Elément de formage de rayons selon la revendication 1,
**caractérisé en ce que**
la réflectivité de la métallisation en couche mince pour le rayonnement optique (4) diminue en permanence du fait de la réaction.

3. Elément de formage de rayons selon la revendication 1 ou 2,
qui est réalisé pour une longueur d'onde du rayonnement optique (4) de 193 nm.

4. Elément de formage de rayons selon une des revendications 1 à 3,
**caractérisé en ce que**
le substrat porteur (1) est composé de verre de quartz.

5. Elément de formage de rayons selon une des revendications 1 à 4,
**caractérisé en ce que**
la métallisation en couche mince est structurée pour former un diaphragme optique pour le rayonnement optique incident (4).

6. Procédé de fabrication d'un élément de formage de rayons pour rayonnement optique, comprenant un substrat porteur (1) comportant une métallisation en couche mince et qui est composé de deux couches métalliques (2, 3), selon les revendications précédentes du brevet, par au moins les étapes suivantes :
- application d'une couche inférieure (2) en Cr sur un substrat porteur (1) transparent au rayonnement optique (4),
- application d'une couche supérieure (3) en Au sur la couche inférieure (2), la couche supérieure (3) en Au étant appliquée dans une épaisseur de 100 nm et la couche inférieure (2) en Cr dans une une épaisseur de 40 mm, l'épaisseur de couche globale de la métallisation en couche mince s'élevant à 140 nm,
- microstructuration de la couche supérieure (3) au moyen d'une photolithographie et d'un processus consécutif d'attaque à sec, et
- microstructuration de la couche inférieure (3) par procédé d'attaque humide, lors duquel la couche supérieure (3) sert de masque d'attaque.

7. Procédé selon la revendication 6,
**caractérisé en ce que**
la couche inférieure (2) et la couche supérieure (3) sont appliquées par un procédé de pulvérisation physique.
